(19) **Europäisches Patentamt / European Patent Office / Office européen des brevets**

(11) **EP 4 442 230 A1**

(12) **EUROPEAN PATENT APPLICATION**
published in accordance with Art. 153(4) EPC

(43) Date of publication:
09.10.2024 Bulletin 2024/41

(21) Application number: 22901277.8

(22) Date of filing: 29.11.2022

(51) International Patent Classification (IPC):
$A61F\ 13/15^{(2006.01)}$      $A61F\ 13/53^{(2006.01)}$

(52) Cooperative Patent Classification (CPC):
A61F 13/15; A61F 13/53

(86) International application number:
PCT/JP2022/043885

(87) International publication number:
WO 2023/100845 (08.06.2023 Gazette 2023/23)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 30.11.2021 JP 2021194706

(71) Applicant: Sumitomo Seika Chemicals Co., Ltd.
Kako-gun, Hyogo 675-0145 (JP)

(72) Inventor: YAMAMOTO, Tomoe
Himeji-shi, Hyogo 672-8076 (JP)

(74) Representative: Mewburn Ellis LLP
Aurora Building
Counterslip
Bristol BS1 6BX (GB)

(54) **ABSORBENT ARTICLE**

(57) Provided is an absorbent article comprising water-absorbent resin particles and an activated carbon, which has an excellent deodorizing effect. An absorbent article comprising at least a liquid-permeable sheet; a liquid-impermeable sheet; and an absorbent material comprising water-absorbent resin particles and an activated carbon, disposed between the liquid-permeable sheet and the liquid-impermeable sheet, wherein the absorbent material comprises water-absorbent resin particles, and the water-absorbent resin particles have a heat release rate of 0.10°C/sec or more and 1.00°C/sec or less as measured by the following method of measuring the heat release rate:
(Method of Measuring the Heat Release Rate)

50 g of physiological saline at 24.9°C is added into a 300 mL volume, cylindrical thermally insulated container and stirred at 600 rpm;
while stirring the physiological saline, 10 g of the water-absorbent resin particles are further added into the thermally insulated container, and the thermally insulated container is closed with a cork stopper having a thermometer; and
a time required for the temperature of the physiological saline to increase by 2.0°C after the addition of the water-absorbent resin particles is measured, and a value of heat release temperature per second is calculated as the heat release rate.

EP 4 442 230 A1

**Description**

Technical Field

The present invention relates to absorbent articles; and more particularly relates to an absorbent article suitably used as hygienic materials, such as disposable diapers, sanitary napkins, and incontinence pads.

Background Art

In recent years, absorbent articles obtained using water-absorbent resins have been widely used in the field of hygienic materials, such as disposable diapers, sanitary napkins, and incontinence pads.

As water-absorbent resins, crosslinked products of partially neutralized salt polymers of acrylic acid have been proposed as preferable water-absorbent resins, because they have many advantages. For example, these water-absorbent resins have good water-absorption capacity; acrylic acid used as a raw material is readily industrially available, and thus, they can be produced at low cost with uniform quality; additionally, they are resistant to decomposition or degradation.

An absorbent article, such as a disposable diaper, a sanitary napkin, or an incontinence pad, is composed of an absorbent material that absorbs and retains a body liquid, such as urine or menses excreted from the body, the absorbent material being positioned mainly in a central portion, a liquid-permeable front sheet (top sheet) positioned on the side of the absorbent article that is brought into contact with the body, and a liquid-impermeable rear sheet (back sheet) positioned opposite to the side that is brought into contact with the body. The absorbent material is typically composed of water-absorbent resin particles and hydrophilic fibers, such as pulp.

When such an absorbent material is used in, for example, a hygienic material, an unpleasant odor such as acetaldehyde may be produced from the absorbent material that has absorbed a body fluid, particularly urine, blood, sweat, or the like.

One known method for reducing such an unpleasant odor is, for example, to add an organic amine compound such as a hydrazide compound to water-absorbent resin particles as an adsorbent for aldehyde compounds (see Patent Literature 1).

Citation List

Patent Literature

Patent Literature 1: JP-A-2001-323155

Summary of Invention

Technical Problem

As described above, one known method for reducing the unpleasant odor produced from the absorbent material is, for example, to use an organic amine compound as an adsorbent for acetaldehyde.

On the other hand, activated carbons are known as adsorbents having an excellent deodorizing effect against a wide range of odors. Thus, the present inventor combined water-absorbent resin particles with an activated carbon in an attempt to impart the deodorizing effect to an absorbent article.

It is a main object of the present invention to provide an absorbent article comprising water-absorbent resin particles and an activated carbon, which has an excellent deodorizing effect.

Solution to Problem

The present inventor has conducted extensive research to solve the aforementioned problem. As a result, the inventor has found that when an absorbent article is produced by disposing an absorbent material comprising water-absorbent resin particles and an activated carbon between a liquid-permeable sheet and a liquid-impermeable sheet, wherein the water-absorbent resin particles have a predetermined heat release rate, the absorbent article can exhibit an improved deodorizing effect. The present invention has been accomplished as a result of further research based on these findings.

In summary, the present invention provides aspects of the invention comprising the following features:

Item 1. An absorbent article comprising at least a liquid-permeable sheet; a liquid-impermeable sheet; and an absorbent material and an activated carbon disposed between the liquid-permeable sheet and the liquid-impermeable sheet,

wherein the absorbent material comprises water-absorbent resin particles, and
the water-absorbent resin particles have a heat release rate of 0.10 °C/sec or more and 1.00°C/sec or less as measured by the following method of measuring the heat release rate:
(Method of Measuring the Heat Release Rate)
50 g of physiological saline at 24.9°C is added into a 300 mL volume, cylindrical thermally insulated container and stirred at 600 rpm;
while stirring the physiological saline, 10 g of the water-absorbent resin particles are further added into the thermally insulated container, and the thermally insulated container is closed with a cork stopper having a thermometer; and
a time required for the temperature of the physiological saline to increase by 2.0°C after the addition of the water-absorbent resin particles is measured, and a value of heat release temperature per second is calculated as the heat release rate.

Item 2. The absorbent article according to item 1, wherein when the absorbent article is viewed in plan view, the activated carbon has a basis weight per unit area of 0.001 g/m$^2$ or more and 6.000 g/m$^2$ or less in a region where the activated carbon is contained.

Item 3. The absorbent article according to item 1, wherein when the absorbent article is viewed in plan view, the activated carbon has a basis weight per unit area of 0.025 g/m$^2$ or more and 7.000 g/m$^2$ or less in that region.

Item 4. The absorbent article according to any one of items 1 to 3, wherein the activated carbon has a median particle size of 1 $\mu$m or more and 100 $\mu$m or less.

Advantageous Effects of Invention

According to the present invention, it is possible to provide an absorbent article comprising water-absorbent resin particles and an activated carbon, which has an excellent deodorizing effect.

Brief Description of Drawings

Fig. 1 is one exemplary schematic cross-sectional view of an absorbent article.
Fig. 2 is a schematic diagram of a measurement apparatus for physiological saline absorption amount under a load of 4.14 kPa.

Description of Embodiments

As used herein, the term "comprising" includes "consisting essentially of" and "consisting of". As used herein, the term "(meth)acrylic" refers to "acrylic or methacrylic", and the term "(meth)acrylate" refers to "acrylate or methacrylate". As used herein, the term "water-soluble" refers to having a water solubility of 5% by mass or more at 25°C.

As used herein, values connected with "to" refer to the numerical range including the values before and after "to" as the lower and upper limits. When a plurality of lower limits and a plurality of upper limits are mentioned separately, any lower limit and any upper limit may be selected and connected with "to".

An absorbent article of the present invention comprises an absorbent material and an activated carbon disposed between a liquid-permeable sheet and a liquid-impermeable sheet. The liquid-permeable sheet is disposed in an outermost portion of the side where the liquid to be absorbed enters. The liquid-impermeable sheet is disposed in an outermost portion of the side opposite to the side where the liquid to be absorbed enters. In addition to the liquid-permeable sheet, the absorbent material, the activated carbon, and the liquid-impermeable sheet, the absorbent article of the present invention may optionally include a core wrap or the like for retaining the shape of the absorbent material. Examples of the absorbent article include diapers (such as disposable diapers), training pants for toilets, incontinence pads, hygienic materials (such as sanitary napkins and tampons), perspiration pads, pet sheets, and animal excrement disposal materials. The absorbent article may be disposable.

In the absorbent article of the present invention, the absorbent material and the activated carbon are disposed between the liquid-permeable sheet and the liquid-impermeable sheet, and the absorbent material comprises water-absorbent resin particles having a heat release rate of 0.10°C/sec or more and 1.00°C/sec or less as measured by the following method of measuring the heat release rate. Because of these features, the absorbent article of the present invention exhibits an excellent deodorizing effect. The water-absorbent resin particles will be later specifically described.

(Method of Measuring the Heat Release Rate)

50 g of physiological saline at 24.9°C is added into a 300 mL volume, cylindrical thermally insulated container and stirred at 600 rpm;

while stirring the physiological saline, 10 g of the water-absorbent resin particles are further added into the thermally insulated container, and the thermally insulated container is closed with a cork stopper having a thermometer; and

a time required for the temperature of the physiological saline to increase by 2.0°C after the addition of the water-absorbent resin particles is measured, and a value of heat release temperature per second is calculated as the heat release rate.

In the present invention, it is only required that the absorbent material and the activated carbon are disposed between the liquid-permeable sheet and the liquid-impermeable sheet. For example, the activated carbon may be disposed as a mixture with the water-absorbent resin particles in the absorbent material. Alternatively, the activated carbon may be disposed between the absorbent material and the liquid-permeable sheet. Alternatively, the activated carbon may be disposed between the absorbent material and the liquid-impermeable sheet. The activated carbon may be disposed in at least one of these locations.

In view of more satisfactorily achieving the effects of the present invention, when the absorbent article of the present invention is viewed in plan view, the activated carbon has a basis weight per unit area of $0.001 \text{ g/m}^2$ or more, more preferably $0.010 \text{ g/m}^2$ or more, still more preferably $0.030 \text{ g/m}^2$ or more, in a region where the activated carbon is contained. On the other hand, the basis weight per unit area of the activated carbon is preferably $6.000 \text{ g/m}^2$ or less, more preferably $3.000 \text{ g/m}^2$ or less, and still more preferably $1.000 \text{ g/m}^2$ or less. A preferred range is from 0.001 to 6.000 $\text{g/m}^2$, a more preferred range is from 0.010 to 3.0000 $\text{g/m}^2$, and a still more preferred range is from 0.030 to 1.000 $\text{g/m}^2$.

As used herein, the phrase "the absorbent article is viewed in plan view" means that the absorbent article is spread on a horizontal plate so as not to create sagging or overlapping and fixed at four sides, and the view is looking down on the absorbent article straight from above in a vertical direction to the horizontal plane. When the absorbent article is of the pant-type, it is difficult to fix the absorbent article at four sides in a spread state; thus, the absorbent article is cut at the boundary between the front body and the back body and then spread on the horizontal plate and fixed at four sides. The area of the region where the activated carbon is contained may be the area of the liquid-permeable sheet, the liquid-impermeable sheet, or other member that can include the activated carbon. In view of ease of calculating the area, the area of the region where the activated carbon is contained may be the area of the liquid-permeable sheet.

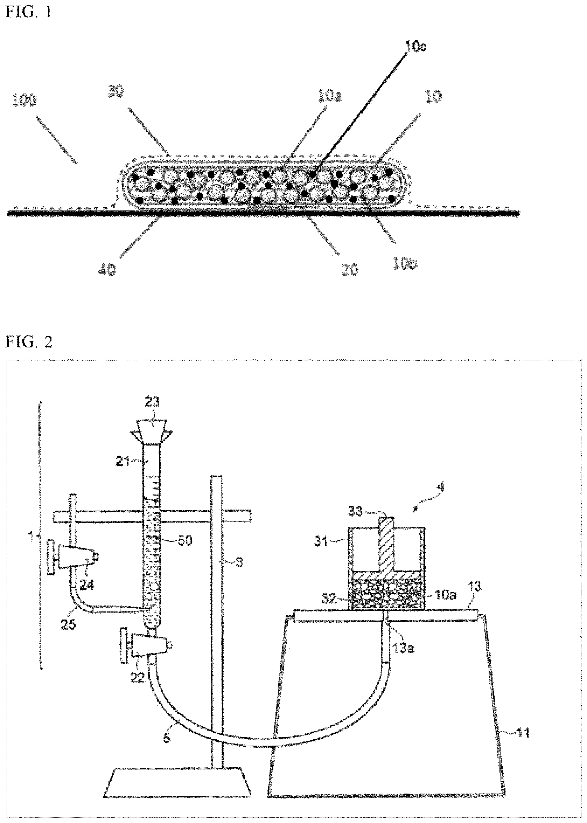

Fig. 1 is a cross-sectional view showing an example of an absorbent article 100. The absorbent article 100 shown in Fig. 1 includes an absorbent material 10, a core wrap 20, a liquid-permeable sheet 30, and a liquid-impermeable sheet 40. In the absorbent article 100, the liquid-impermeable sheet 40, the absorbent material 10 with an outer periphery covered by the core wrap 20, and the liquid-permeable sheet 30 are laminated in this order. Fig. 1 shows a schematic diagram of a case where water-absorbent resin particles 10a and an activated carbon 10c are contained in the absorbent material 10.

The absorbent material 10 includes the water-absorbent resin particles 10a and a fiber layer 10b containing hydrophilic fibers which is preferably used. The absorbent material 10 may or may not contain hydrophilic fibers. The water-absorbent resin particles 10a are, for example, supported on and dispersed in the fiber layer 10b. Examples of the structure of the absorbent material 10 include a sheet-like structure in which the water-absorbent resin particles are fixed on a nonwoven fabric or between a plurality of nonwoven fabrics; a mixed dispersion obtained by mixing the water-absorbent resin particles and hydrophilic fibers to give a homogeneous composition; a sandwich structure in which the water-absorbent resin particles are sandwiched between layered hydrophilic fibers obtained by, for example, pressing pulverized hydrophilic fibers; and a structure in which the water-absorbent resin particles and hydrophilic fibers are wrapped in a tissue.

The planar shape of the absorbent material 10 may be determined appropriately according to its use or according to the shape of the absorbent article. Examples of the planar shape include a substantially rectangular shape, an oval shape, an hourglass shape, and a battledore shape. The absorbent material may have a slit or the like for improved fit. The internal structure of the absorbent material 10 may also be determined appropriately according to its purpose. Examples include, in addition to a structure formed of a single absorbent material, a structure in which a plurality of absorbent materials are combined (for example, divided on the plane, or divided vertically); and a structure in which the inside of the absorbent material has a quantitative distribution gradient (such as a uniform distribution or a quantitative distribution according to the portion where a liquid is to be added) of the water-absorbent resin particles and other components.

The water-absorbent resin particles in the absorbent material 10 have a basis weight of, for example, 50 g/m$^2$ or more and 700 g/m$^2$ or less. In view of more satisfactorily achieving the effects of the present invention, the basis weight is preferably 100 g/m$^2$ or more, more preferably 120 g/m$^2$ or more, and still more preferably 140 g/m$^2$ or more, while it is preferably 600 g/m$^2$ or less, more preferably 500 g/m$^2$ or less, and still more preferably 400 g/m$^2$ or less.

The water-absorbent resin particles content in the absorbent material is preferably 5 to 100% by mass, more preferably 10 to 95% by mass, still more preferably 20 to 90% by mass, and even more preferably 30 to 80% by mass.

Examples of the hydrophilic fibers include at least one selected from the group consisting of pulverized wood pulp, cotton, cotton linter, rayon, cellulose acetate, polyamides, polyesters, and polyolefins. More specifically, examples include cellulose fibers, such as cotton-like pulp made from wood, mechanical pulp, chemical pulp, and semi-chemical pulp; artificial cellulose fibers, such as rayon and acetate; and hydrophilized fibers made of synthetic resins, such as polyamides, polyesters, and polyolefins. The hydrophilic fibers typically have an average fiber length of 0.1 to 10 mm. Alternatively, the average fiber length may be 0.5 to 5 mm.

The absorbent material may further contain additives such as inorganic powders (for example, amorphous silica) other than the activated carbon, deodorants, pigments, dyes, anti-bacterial agents, perfumes, and adhesives. These additives can impart various functions to the absorbent material. When the water-absorbent resin particles contain inorganic particles, the absorbent material may contain an inorganic powder separately from the inorganic particles in the water-absorbent resin particles. Examples of the inorganic powder include silicon dioxide, zeolite, kaolin, and clay.

The liquid-permeable sheet 30 is disposed in the outermost portion of the side where the liquid to be absorbed enters. The liquid-permeable sheet 30 is disposed, for example, on the core wrap 20, in contact with the core wrap 20. The absorbent material 10 is sealed in the core wrap 20, so that its shape can be retained. The absorbent material may be blended with an adhesive binder to improve the shape retention before and during use of the absorbent material. The liquid-impermeable sheet 40 is disposed in the outermost portion of the side opposite to the liquid-permeable sheet 30 in the absorbent article 100. The liquid-impermeable sheet 40 is disposed on the lower side of the core wrap 20 in contact with the core wrap 20. The liquid-permeable sheet 30 and the liquid-impermeable sheet 40 have, for example, main surfaces wider than a main surface of the absorbent material 10, and outer edge portions of the liquid-permeable sheet 30 and the liquid-impermeable sheet 40 extend around the absorbent material 10 and the core wrap 20.

The size relationship among the absorbent material 10, the optional core wrap 20, the liquid-permeable sheet 30, and the liquid-impermeable sheet 40 is not specifically limited, and may be adjusted appropriately according to the use of the absorbent article and the like.

The liquid-permeable sheet 30 may be a sheet formed of a resin or fibers commonly used in the art. In view of liquid permeability, flexibility, and strength when used in the absorbent article, the liquid-permeable sheet 30 may contain, for example, synthetic resins, such as polyolefins, for example, polyethylene (PE) and polypropylene (PP), polyesters, for example, polyethylene terephthalate (PET), polytrimethylene terephthalate (PTT), and polyethylene naphthalate (PEN), polyamides, for example, nylon, and rayon, regenerated fibers such as cupro, acetate, or synthetic fibers containing these synthetic resins. The liquid-permeable sheet 30 may also be formed of natural fibers including cotton, silk, hemp, or pulp (cellulose). In view of increasing the strength of the liquid-permeable sheet 30, the liquid permeable-sheet 30 may contain synthetic fibers. In particular, the synthetic fibers may be polyolefin fibers, polyester fibers, or a combination thereof. These materials may be used alone or in combinations of two or more.

The liquid-permeable sheet 30 may be a nonwoven fabric, a porous sheet, or a combination thereof. The nonwoven fabric is a sheet obtained by intertwining fibers without weaving. The nonwoven fabric may be a nonwoven fabric composed of short fibers (i.e., staple) (short fiber nonwoven fabric) or a nonwoven fabric composed of long fibers (i.e., filament) (long fiber nonwoven fabric). The staple may generally have a fiber length of, but not limited to, several hundred mm or less.

The liquid-permeable sheet 30 may be a thermally bonded nonwoven fabric, an air-through nonwoven fabric, a resin-bonded nonwoven fabric, a spunbond nonwoven fabric, a meltblown nonwoven fabric, an air-laid nonwoven fabric, a spunlace nonwoven fabric, a point-bonded nonwoven fabric, or a laminate of two or more types of nonwoven fabrics selected from the above. These nonwoven fabrics can be formed of, for example, the above-mentioned synthetic fibers or natural fibers. The laminate of two or more types of nonwoven fabrics may be, for example, a spunbond/meltblown/spunbond nonwoven fabric, which is a composite nonwoven fabric having a spunbond nonwoven fabric, a meltblown nonwoven fabric, and a spunbond nonwoven fabric laminated in this order. In view of preventing liquid leakage, the liquid-permeable sheet 30 may be a thermally bonded nonwoven fabric, an air-through nonwoven fabric, a spunbond nonwoven fabric, or a spunbond/meltblown/spunbond nonwoven fabric.

The nonwoven fabric used as the liquid-permeable sheet 30 desirably has appropriate hydrophilicity, in view of the liquid absorption performance of the absorbent article. From this viewpoint, the liquid-permeable sheet 30 may be a nonwoven fabric having a degree of hydrophilicity of 5 to 200 as measured according to the measurement method of pulp and paper test method No. 68 (2000) by the Japan Technical Association of the Pulp and Paper Industry. The degree of hydrophilicity of the nonwoven fabric may be 10 to 150. For details of the pulp and paper test method No. 68, reference may be made, for example, to WO 2011/086843.

The nonwoven fabric having hydrophilicity as described above may be formed of fibers exhibiting an appropriate degree of hydrophilicity, such as rayon fibers, or may be formed of fibers obtained by hydrophilizing hydrophobic chemical fibers, such as polyolefin fibers and polyester fibers. Examples of methods for obtaining nonwoven fabrics containing hydrophilized hydrophobic chemical fibers include a method in which a nonwoven fabric is obtained by a spunbonding method using a mixture of hydrophobic chemical fibers and a hydrophilizing agent; a method in which a spunbond nonwoven fabric is produced using hydrophobic chemical fibers with a hydrophilizing agent; and a method in which a spunbond nonwoven fabric obtained using hydrophobic chemical fibers is impregnated with a hydrophilizing agent. Examples of usable hydrophilizing agents include anionic surfactants, such as aliphatic sulfonates and higher alcohol sulfates; cationic surfactants, such as quaternary ammonium salts; nonionic surfactants, such as polyethylene glycol fatty acid esters, polyglycerol fatty acid esters, and sorbitan fatty acid esters; silicone surfactants, such as polyoxyalkylene-modified silicones; and stain-release agents composed of polyester, polyamide, acrylic, and urethane resins.

The liquid-permeable sheet 30 may be a nonwoven fabric that is moderately bulky and has a large basis weight, in view of imparting good liquid permeability, flexibility, strength, and cushioning properties to the absorbent article, and in view of increasing the liquid permeation rate of the absorbent article. The nonwoven fabric used as the liquid-permeable sheet 30 may have a basis weight of 5 to 200 $g/m^2$, 8 to 150 $g/m^2$, or 10 to 100 $g/m^2$. The nonwoven fabric used as the liquid-permeable sheet 30 may have a thickness of 20 to 1400 $\mu$m, 50 to 1200 $\mu$m, or 80 to 1000 $\mu$m.

Furthermore, the liquid-permeable sheet 30 may be composed of two or more sheets, and may have an embossed or perforated surface, in order to improve the liquid diffusion properties. Embossing or perforation may be performed using a known method. The liquid-permeable sheet 30 may also contain skin lotions, moisturizers, anti-oxidizing agents, anti-inflammatory agents, pH adjusters, and the like, in order to reduce skin irritation. The liquid-permeable sheet 30 may have a shape that covers the absorbent material 10, so as to prevent liquid leakage, although the shape may also depend on the shapes of the absorbent material and the absorbent article.

The core wrap 20 is, for example, disposed to cover the outer periphery of the absorbent material 10. The absorbent material 10 is disposed in the core wrap 20. Examples of the core wrap 20 include tissues and nonwoven fabrics. The core wrap 20 has, for example, a main surface equal in size to the main surface of the absorbent material 10. The absorbent material 10 is sealed in the core wrap 20, so that its shape is retained. Methods for retaining the shape of the absorbent material with the core wrap are not limited thereto; for example, the absorbent material may be sandwiched between two individual upper and lower core wraps; or the core wrap may form a bag in which the absorbent material is disposed.

The absorbent material may be blended with an adhesive binder to improve the shape retention before and during use of the absorbent material. Examples of the adhesive binder include thermal bonding synthetic fibers, hot melt adhesives, and adhesive emulsions.

Examples of thermal bonding synthetic fibers include total fusion-type binders, such as polyethylene, polypropylene, and ethylene-propylene copolymers; and a non-total fusion-type binder having a side-by-side or core-in-sheath structure of polypropylene and polyethylene. In the non-total fusion-type binder, only the polyethylene portion is thermal-bonded. Examples of hot melt adhesives include blends of base polymers, such as ethylene-vinyl acetate copolymers, styrene-isoprene-styrene block copolymers, styrene-butadiene-styrene block copolymers, styrene-ethylene-butylene-styrene block copolymers, styrene-ethylene-propylene-styrene block copolymers, and amorphous polypropylene, with tackifiers, plasticizers, antioxidants, and the like.

Examples of adhesive emulsions include polymers of at least one monomer selected from the group consisting of methyl methacrylate, styrene, acrylonitrile, 2-ethylhexyl acrylate, butyl acrylate, butadiene, ethylene, and vinyl acetate. These adhesive binders may be used alone or in combinations of two or more.

The liquid-impermeable sheet 40 is disposed in the outermost portion of the side opposite to the liquid-permeable sheet 30 in the absorbent article 100. The liquid-impermeable sheet 40 is disposed on the lower side of the core wrap 20 in contact with the core wrap 20. The liquid-impermeable sheet 40 has, for example, a main surface wider than the main surface of the absorbent material 10, and the outer edge portion of the liquid-impermeable sheet 40 extends around the absorbent material 10 and the core wrap 20. The liquid-impermeable sheet 40 prevents the liquid absorbed by the absorbent material 10 from leaking out through the liquid-impermeable sheet 40 side.

Examples of the liquid-impermeable sheet 40 include a sheet made of a synthetic resin, such as polyethylene, polypropylene, or polyvinyl chloride; a sheet made of a nonwoven fabric, such as a spunbond/meltblown/spunbond (SMS) nonwoven fabric in which a water-resistant meltblown nonwoven fabric is sandwiched between high-strength spunbond nonwoven fabrics; and a sheet made of a composite material of these synthetic resins and a nonwoven fabric (for example, a spunbond nonwoven fabric or a spunlace nonwoven fabric). The liquid-impermeable sheet 40 may have breathability, in view of reducing dampness during wearing to reduce discomfort to the wearer. The liquid-impermeable sheet 40 may be a sheet made of a synthetic resin mainly composed of a low-density polyethylene (LDPE) resin. In view of ensuring flexibility so as not to impair the wearing comfort of the absorbent article, the liquid-impermeable sheet 40 may be a sheet made of a synthetic resin having a basis weight of 10 to 50 $g/m^2$, for example. To impart breathability to the liquid-impermeable sheet 40, for example, the resin sheet may be blended with a filler, or the liquid-impermeable sheet 40 may be embossed. The filler may be, for example, calcium carbonate.

In addition to the above-described liquid-permeable sheet, absorbent material, liquid-impermeable sheet, and core wrap, the absorbent article 100 may include other members, as required, according to its use and function. Examples of such members include an outer cover nonwoven fabric and leg gathers.

(Outer Cover Nonwoven Fabric)

An outer cover nonwoven fabric may be disposed on the side of the liquid-impermeable sheet 40 opposite to the absorbent material. The outer cover nonwoven fabric may be bonded to the liquid-impermeable sheet 40 with an adhesive, for example. The outer cover nonwoven fabric may be formed of one or more layers and may be a soft material. To appeal to the consumer's desire to purchase or for other reasons, the outer cover nonwoven fabric may have a flexible feel; may have a pattern printed; or may have a plurality of bonding portions formed thereon, or may be embossed, or may have a three-dimensional shape formed thereon.

(Leg Gathers)

The absorbent article 100 of the present invention may include leg gathers including an elastic member with stretch properties, disposed outside both ends in the width direction of the absorbent material 10 and placed substantially parallel to the longitudinal direction of the absorbent material 10. The length of the leg gathers is adjusted to be approximately equal to or longer than the wearer's leg circumference. The elongation rate of the leg gathers is adjusted appropriately, in view of reducing tight feeling when worn for a long period of time while preventing leakage of the discharged liquid.

(Front/Back Gathers)

The absorbent article 100 of the present invention may include front/back gathers, which are disposed near both ends in the longitudinal direction of the absorbent article and include an elastic member that stretches in the width direction.

The absorbent article 100 includes the front/back gathers that can stand above side edge portions in the width direction of the absorbent material 10. More specifically, on each of both sides in the longitudinal direction of the absorbent article, a sheet member of the front/back gathers having a gather elastic member is disposed to constitute the front/back gathers.

The members of the front/back gathers are typically made of a liquid-impermeable or water-repellent material, which is preferably a moisture-permeable material. Examples of the material include a liquid-impermeable or water-repellent porous sheet, a liquid-impermeable or water-repellent nonwoven fabric, and a laminate of the porous sheet and the nonwoven fabric. Examples of the nonwoven fabric include thermal bonded nonwoven fabrics, spunbond nonwoven fabrics, meltblown nonwoven fabrics, spunlace nonwoven fabrics, and spunbond/meltblown/spunbond nonwoven fabrics. The above-described members may have a basis weight of 5 to 100 g/m$^2$, 8 to 70 g/m$^2$, or 10 to 40 g/m$^2$.

The absorbent article 100 can be produced, for example, using a method including disposing the absorbent material 10 in the core wrap 20, and disposing these members between the liquid-permeable sheet 30 and the liquid-impermeable sheet 40. The laminate of the liquid-impermeable sheet 40, the core wrap 20, the absorbent material 10, the core wrap 20, and the liquid-permeable sheet 30 laminated in this order is optionally pressurized. The laminate may or may not include the liquid-impermeable sheet 40.

The members constituting the absorbent article 100 may be bonded to one another. For example, the absorbent material 10 and the liquid-permeable sheet 30 may be bonded, which allows a liquid to be more smoothly guided to the absorbent material and facilitates obtaining an absorbent article with excellent leakage proofness. When the absorbent material 10 is covered by the core wrap 20 or sandwiched between the core wraps 20, it is preferred that at least the core wrap 20 and the liquid-permeable sheet 30 are bonded, and more preferred that the core wrap 20 and the absorbent material 10 are also bonded. Examples of bonding methods include adhesives, heat sealing, ultrasonic sealing, and other known methods. Examples include a method in which a hot melt adhesive is applied to the liquid-permeable sheet 30 in the form of longitudinal stripes, spirals, or the like at a predetermined spacing in the width direction; and a method using a water-soluble adhesive selected from starch, carboxymethylcellulose, polyvinyl alcohol, polyvinylpyrrolidone, and other water-soluble polymers. When the absorbent material 10 contains thermal bonding synthetic fibers, a method that utilizes the thermal bonding may be employed.

The shape of the absorbent article 100 may be determined appropriately according to the use. For example, when the absorbent article is a urine pad for light incontinence or a sanitary napkin, examples of the shape include a substantially rectangular shape, an oval shape, an hourglass shape, and a battledore shape.

The absorbent article of the present invention preferably includes a liquid permeability improving means in at least one of the liquid-permeable sheet, the absorbent material, and between the liquid-permeable sheet and the absorbent material. The liquid permeability improving means is a means provided for improving the permeability of the liquid from the liquid-permeable sheet to the absorbent material, and examples include means such as an embossed structure, a slit structure, and disposition of a liquid acquisition/diffusion sheet. The liquid permeability improving means is preferably the embossed structure and/or the disposition of the liquid acquisition/diffusion sheet. The liquid permeability improving means can further improve the absorption properties (such as permeation rate and leakage performance) of the absorbent article.

The embossed structure is specifically an uneven shape formed by embossing a surface of the liquid-permeable sheet or the absorbent material. The uneven shape allows the liquid to easily permeate. The pattern of the embossed structure when viewed in plan view is not specifically limited and may be, for example, a grid shape.

The slit structure is a structure in which at least a portion of the absorbent material is provided with a slit-shaped hole. The slit structure may also be a recess, a groove, or a slit structure formed by cutting, compressing, or making a through hole in the absorbent material in the thickness direction. Disposing the slit so that the slit forms a flow path of the liquid facilitates liquid diffusion along the flow path, allowing the liquid to easily permeate. The slit structure can be formed, for example, in a central portion of the absorbent material in a straight line from a midpoint to both ends in the longitudinal direction. When the absorbent material is viewed in plan view, the ratio of the area of the slit structure to the area (100%) of the absorbent material is preferably about 2 to 30%.

The liquid acquisition/diffusion sheet is disposed between the liquid-permeable sheet and the absorbent material. The liquid acquisition/diffusion sheet allows the liquid permeated the liquid-permeable sheet 30 to rapidly move to the absorbent material 10 side. The liquid acquisition/diffusion sheet and the liquid-permeable sheet 30 may be bonded using a hot melt adhesive, or using heat embossing or ultrasonic welding. The liquid acquisition/diffusion sheet may be a nonwoven fabric, or may also be a resin film with numerous permeable holes. While the same materials as those mentioned in the section concerning the liquid-permeable sheet 30 may be used as the nonwoven fabric, it is preferred to use a nonwoven fabric with higher hydrophilicity or with a higher fiber density than that of the liquid-permeable sheet 30, because this achieves improved liquid migration properties toward the direction of the absorbent material.

The liquid acquisition/diffusion sheet is typically disposed in the central portion to be shorter in width than the absorbent material 10, although the liquid acquisition/diffusion sheet may be disposed across the entire width. The length in the front-to-back direction of the liquid acquisition/diffusion sheet may be substantially identical to the entire length of the absorbent article, or may be substantially identical to the entire length of the absorbent material 10, or may be a length contemplated to cover a region where the liquid is to be added. The liquid acquisition/diffusion sheet preferably has a basis weight of 15 to 75 g/m$^2$.

(Activated Carbon)

In view of more satisfactorily achieving the effects of the present invention, the activated carbon preferably has a median particle size of 1 $\mu$m or more, more preferably 5 $\mu$m or more, still more preferably 10 $\mu$m or more. On the other hand, the median particle size is preferably 100 $\mu$m or less, more preferably 70 $\mu$m or less, and still more preferably 50 $\mu$m or less. Preferred ranges include from 1 to 100 $\mu$m and from 5 to 70 $\mu$m.

The median particle size (D50 (median size), volume-based) of the activated carbon can be measured using a laser diffraction particle size distribution analyzer; specifically, it is the value as measured by the method described in the Examples section.

In view of more satisfactorily achieving the effects of the present invention, the activated carbon preferably has a shape such as a crushed shape or a cylindrical shape, more preferably a crushed shape.

In view of more satisfactorily achieving the effects of the present invention, the activated carbon preferably has a BET specific surface area of 100 m$^2$/g or more, more preferably 1000 m$^2$/g or more. On the other hand, the BET specific surface area is preferably 3000 m$^2$/g or less, and more preferably 2000 m$^2$/g or less. Preferred ranges include from 100 to 3000 m$^2$/g and from 1000 to 2000 m$^2$/g.

The BET specific surface area of the activated carbon can be measured using a specific surface area analyzer; specifically, it is the value as measured by the method described in the Examples section.

In view of more satisfactorily achieving the effects of the present invention, the activated carbon is preferably an activated carbon having a polar functional group (hydrophilic functional group) on the surface (i.e., a hydrophilic activated carbon). Examples of polar functional groups include hydroxy, carboxy, and phenol groups. Activated carbons having polar functional groups on the surface are commercially available as, for example, activated carbons for liquid phase applications and activated carbons for water treatment.

The activated carbon may be produced from, for example, coconut shells, infusibilized or carbonized organic materials, and infusible resins such as phenol resins. Examples of the organic materials include polyacrylonitrile, pitch, polyvinyl alcohol, and cellulose. Among these, the activated carbon is preferably produced from coconut shells or pitch (such as coal pitch).

In view of more satisfactorily achieving the effects of the present invention, when the absorbent material is viewed in plan view, the activated carbon preferably has a basis weight per unit area of 0.025 $g/m^2$ or more, more preferably 0.050 $g/m^2$ or more, still more preferably 0.100 $g/m^2$ or more, in that region. On the other hand, the basis weight per unit area of the activated carbon is preferably 7.000 $g/m^2$ or less, more preferably 6.000 $g/m^2$ or less, and still more preferably 5.000 $g/m^2$ or less. A preferred range is from 0.025 to 7.000 $g/m^2$, a more preferred range is from 0.050 to 6.000 $g/m^2$, and a still more preferred range is from 0.100 to 5.000 $g/m^2$.

As used herein, the phrase "the absorbent material is viewed in plan view" means that the absorbent material is allowed to stand on a horizontal plate so as not to create sagging or overlapping, and the view is looking down on the absorbent material straight from above in a vertical direction to the horizontal plane. The term "that region" means a region surrounded with outer edges of the members constituting the absorbent material.

In view of more satisfactorily achieving the effects of the present invention, the amount of the activated carbon contained in the absorbent article of the present invention is preferably 0.0005 part by mass or more, more preferably 0.0010 part by mass or more, and still more preferably 0.0100 part by mass or more, per 100 parts by mass of the absorbent material. On the other hand, the amount of the activated carbon is preferably 1.0000 part by mass or less, more preferably 0.8000 part by mass or less, and still more preferably 0.6000 part by mass or less, or 0.5000 part by mass or less. Preferred ranges include from 0.0005 to 1.0000 part by mass and from 0.0010 to 0.8000 part by mass.

In view of more satisfactorily achieving the effects of the present invention, the amount of the activated carbon contained in the absorbent article of the present invention is preferably 0.05 part by mass or more, more preferably 0.10 part by mass or more, and still more preferably 0.20 part by mass or more, per 100 parts by mass of the water-absorbent resin particles. On the other hand, the amount of the activated carbon is preferably 7.00 parts by mass or less, more preferably 3.00 parts by mass, still more preferably 1.00 part by mass or less, and even more preferably 0.50 part by mass or less. Preferred ranges include from 0.05 to 7.00 parts by mass and from 0.10 to 6.00 parts by mass.

In view of more satisfactorily achieving the effects of the present invention, when the hydrophilic fibers are contained in the absorbent material, the amount of the activated carbon contained in the absorbent article of the present invention is preferably 0.005 part by mass or more, more preferably 0.050 part by mass or more, and still more preferably 0.100 part by mass or more, per 100 parts by mass of the hydrophilic fibers. On the other hand, the amount of the activated carbon is preferably 7.700 parts by mass or less, more preferably 4.000 parts by mass or less, still more preferably 3.000 parts by mass or less, and even more preferably 2.500 parts by mass or less. Preferred ranges include from 0.005 to 7.700 parts by mass and from 0.050 to 4.000 parts by mass.

In view of more satisfactorily achieving the effects of the present invention, the activated carbon preferably has an iodine adsorption amount of 100 mg/g or more, more preferably 500 mg/g or more. On the other hand, the iodine adsorption amount is preferably 3000 mg/g or less, and more preferably 2000 mg/g or less. Preferred ranges include from 100 to 3000 mg/g and from 500 to 2000 mg/g.

As used herein, the iodine adsorption amount of the activated carbon is the value as measured according to JIS K 1474: 2014.

In view of more satisfactorily achieving the effects of the present invention, the activated carbon preferably has a loss on drying of 0.1% or more, more preferably 0.5% or more. On the other hand, the loss on drying is preferably 8% or less, and more preferably 5% or less. Preferred ranges include from 0.1 to 8% and from 0.5 to 5%.

As used herein, the loss on drying of the activated carbon is the value as measured according to JIS K 1474: 2014.

In view of more satisfactorily achieving the effects of the present invention, the activated carbon preferably has a pH of 3 or more, more preferably 5 or more. On the other hand, the pH is preferably 12 or less, and more preferably 11 or less. Preferred ranges include from 3 to 12 and from 5 to 11.

As used herein, the pH of the activated carbon is the value as measured according to JIS K 1474: 2014.

When the activated carbon is contained in the absorbent material, preferably, the activated carbon is disposed on surfaces of the water-absorbent resin particles (i.e., the activated carbon is present on surfaces of the water-absorbent resin particles).

The water-absorbent resin particles contained in the absorbent material will be described in detail next.

(Water-Absorbent Resin Particles)

As described above, in the absorbent article of the present invention, the water-absorbent resin particles contained in the absorbent material have a heat release rate of 0.10°C/sec or more and 1.00°C/sec or less as measured by the method of measuring the heat release rate described above.

The heat release rate of the water-absorbent resin particles is preferably 0.10°C/sec or more, and more preferably 0.15°C/sec or more. On the other hand, the heat release rate of the water-absorbent resin particles is preferably 1.00°C/sec or less, and more preferably 0.60°C/sec or less. Preferred ranges include from 0.10 to 1.00°C/sec and from 0.15 to 0.60°C/sec.

The water-absorbent resin particles are formed of a crosslinked product of a polymer of a water-soluble ethylenically unsaturated monomer, that is, a crosslinked polymer having a structural unit derived from a water-soluble ethylenically unsaturated monomer.

In view of more satisfactorily achieving the effects of the present invention, the water-absorbent resin particles preferably have a water-absorption rate, as measured by a Vortex method, of 60 seconds or less, more preferably 50 seconds or less, still more preferably 40 seconds or less. On the other hand, the water-absorption rate is preferably 1 second or more, more preferably 3 seconds or more, and still more preferably 10 seconds or more. Preferred ranges include from 1 to 60 seconds and from 3 to 40 seconds.

The water-absorption rate of the water-absorbent resin particles as measured by the Vortex method is the value as measured by the method described in the Examples section.

In view of more satisfactorily achieving the effects of the present invention, the water-absorbent resin particles preferably have a physiological saline retention amount of 20 g/g or more, more preferably 25 g/g or more, still more preferably 30 g/g or more. On the other hand, the physiological saline retention amount is preferably 60 g/g or less, more preferably 55 g/g or less, and still more preferably 50 g/g or less, for example.

In view of more satisfactorily achieving the effects of the present invention, the water-absorbent resin particles preferably have a physiological saline absorption amount under a load of 4.14 kPa of 5 mL/g or more, more preferably 10 mL/g or more, still more preferably 15 mL/g or more. On the other hand, the physiological saline absorption amount under a load of 4.14 kPa is preferably 40 mL/g or less, more preferably 35 mL/g or less, and still more preferably 30 mL/g or less. Preferred ranges include from 5 to 40 mL/g.

The physiological saline retention amount and the physiological saline absorption amount under a load of 4.14 kPa of the water-absorbent resin particles are each the value as measured by the method described in the Examples section.

In view of more satisfactorily achieving the effects of the present invention, the water-absorbent resin particles preferably have a BET specific surface area of 0.01 m$^2$/g or more, more preferably 0.02 m$^2$/g or more, still more preferably 0.04 m$^2$/g or more. On the other hand, the BET specific surface area is preferably 0.20 m$^2$/g or less, more preferably 0.17 m$^2$/g or less, and still more preferably 0.15 m$^2$/g or less. Preferred ranges include from 0.01 to 0.20 m$^2$/g, from 0.02 to 0.17 m$^2$/g, and from 0.04 to 0.15 m2/g.

The BET specific surface area of the water-absorbent resin particles can be measured using a specific surface area analyzer; specifically, it is the value as measured by the method described in the Examples section.

The water-absorbent resin is typically particulate. In view of satisfactorily achieving the effects of the present invention while avoiding local absorption in the absorbent article, the water-absorbent resin particles preferably have a median particle size of 150 $\mu$m or more, 200 $\mu$m or more, 240 $\mu$m or more, 260 $\mu$m or more, 280 $\mu$m or more, or 300 $\mu$m or more. On the other hand, in view of satisfactorily achieving the effects of the present invention while imparting a comfortable feel to the absorbent article, the median particle size is preferably 850 $\mu$m or less, 600 $\mu$m or less, 550 $\mu$m or less, 500 $\mu$m or less, 450 $\mu$m or less, or 400 $\mu$m or less. That is, the median particle size is preferably 150 to 850 $\mu$m, more preferably 200 to 600 $\mu$m, still more preferably 240 to 500 $\mu$m, even more preferably 280 to 450 $\mu$m, and still more preferably 300 to 400 $\mu$m.

The water-absorbent resin particles may be in the form of particles each composed of a single particle, or in the form of particles (secondary particles) formed by agglomeration of fine particles (primary particles). Examples of the shape of the primary particles include a substantially spherical shape, a crushed indefinite shape, and a flat shape. When the water-absorbent resin particles are primary particles produced by reversed phase suspension polymerization, examples of the shape include a substantially spherical single-particle shape with a smooth surface shape, such as a spherical shape or an elliptical sphere shape.

The median particle size of the water-absorbent resin particles can be measured using JIS standard sieves; specifically, it is the value as measured by the method described in the Examples section.

A representative polymerization method, such as aqueous polymerization, emulsion polymerization, or reversed phase suspension polymerization, is used for polymerizing the water-soluble ethylenically unsaturated monomer. In aqueous polymerization, polymerization is performed by heating, optionally with stirring, an aqueous solution of the water-soluble ethylenically unsaturated monomer. In reversed phase suspension polymerization, polymerization is performed by heating the water-soluble ethylenically unsaturated monomer with stirring in a hydrocarbon dispersion medium.

One exemplary method for producing the water-absorbent resin particles will be hereinafter described.

Specific examples of methods for producing the water-absorbent resin particles include a method for producing the water-absorbent resin particles by performing reversed phase suspension polymerization of the water-soluble ethylenically unsaturated monomer in a hydrocarbon dispersion medium, the method including the steps of performing the polymerization in the presence of a radical polymerization initiator; and surface-crosslinking a hydrous gel obtained by the polymerization, in the presence of a surface-crosslinking agent. In the method for producing the water-absorbent resin particles of the present invention, an internal-crosslinking agent may be optionally added to the water-soluble ethylenically unsaturated monomer to obtain a hydrous gel having an internal-crosslinked structure.

<Polymerization Step>

[Water-Soluble Ethylenically Unsaturated Monomer]

Examples of the water-soluble ethylenically unsaturated monomer include (meth)acrylic acid ("acryl" and "methacryl" are herein collectively referred to as "(meth)acryl"; the same applies below) and salts thereof; 2-(meth)acrylamido-2-methylpropanesulfonic acid and salts thereof; nonionic monomers, such as (meth)acrylamide, N,N-dimethyl(meth)acrylamide, 2-hydroxyethyl(meth)acrylate, N-methylol(meth)acrylamide, and polyethylene glycol mono(meth)acrylate; and amino group-containing unsaturated monomers, such as N,N-diethylaminoethyl (meth)acrylate, N,N-diethylaminopropyl (meth)acrylate, and diethylaminopropyl (meth)acrylamide, as well as quaternary compounds thereof. Preferred among these water-soluble ethylenically unsaturated monomers are (meth)acrylic acid and salts thereof, (meth)acrylamide, and N,N-dimethylacrylamide, and more preferred are (meth)acrylic acid and salts thereof, because they are readily industrially available, for example. These water-soluble ethylenically unsaturated monomers may be used alone or in combinations of two or more.

Among these water-soluble ethylenically unsaturated monomers, acrylic acid and salts thereof are widely used as raw materials of water-absorbent resin particles. Copolymers of acrylic acid and/or salts thereof with other water-soluble ethylenically unsaturated monomers as mentioned above may also be used. In this case, acrylic acid and/or a salt thereof as a main water-soluble ethylenically unsaturated monomer is preferably used in an amount of 70 to 100 mol% based on the total amount of water-soluble ethylenically unsaturated monomers.

The water-soluble ethylenically unsaturated monomer may be dispersed as an aqueous solution in a hydrocarbon dispersion medium and then subjected to reversed phase suspension polymerization. When the water-soluble ethylenically unsaturated monomer is in the form of an aqueous solution, the dispersion efficiency in the hydrocarbon dispersion medium can be increased. The concentration of the water-soluble ethylenically unsaturated monomer in the aqueous solution is preferably in the range of 20% by mass to not more than the saturation concentration. The concentration of the water-soluble ethylenically unsaturated monomer is more preferably 55% by mass or less, still more preferably 50% by mass or less, and even more preferably 45% by mass or less. On the other hand, the concentration of the water-soluble ethylenically unsaturated monomer is more preferably 25% by mass or more, still more preferably 28% by mass or more, and even more preferably 30% by mass or more.

When the water-soluble ethylenically unsaturated monomer has an acid group such as (meth)acrylic acid or 2-(meth)acrylamido-2-methylpropanesulfonic acid, the acid group may be optionally neutralized with an alkaline neutralizing agent, before the water-soluble ethylenically unsaturated monomer is used. Examples of such alkaline neutralizing agents include alkali metal salts, such as sodium hydroxide, sodium carbonate, sodium hydrogen carbonate, potassium hydroxide, and potassium carbonate; and ammonia. These alkaline neutralizing agents may be used in the form of an aqueous solution to facilitate the neutralization operation. The above-mentioned alkaline neutralizing agents may be used alone or in combinations of two or more.

The degree of neutralization of the water-soluble ethylenically unsaturated monomer with an alkaline neutralizing agent, calculated as the degree of neutralization of all acid groups in the water-soluble ethylenically unsaturated monomer, is preferably 10 to 100 mol%, more preferably 30 to 90 mol%, still more preferably 40 to 85 mol%, and even more preferably 50 to 80 mol%.

[Radical Polymerization Initiator]

Examples of the radical polymerization initiator to be added in the polymerization step include persulfates, such as potassium persulfate, ammonium persulfate, and sodium persulfate; peroxides, such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide; and azo compounds, such as 2,2'-azobis(2-amidinopropane) dihydrochloride, 2,2'-azobis[2-(N-phenylamidino)propane] dihydrochloride, 2,2'-azobis[2-(N-allylamidino)propane] dihydrochloride, 2,2'-azobis{2-[1-(2-hydroxyethyl)-2-imidazolin-2-yl]propane} dihydrochloride, 2,2'-azobis{2-methyl-N-[1,1-bis(hydroxymethyl)-2-hydroxyethyl]propionamide}, 2,2'-azobis[2-methyl-N-(2-hydroxyethyl)-propionamide], and 4,4'-azobis(4-cyanovaleric acid). Preferred among these radical polymerization initiators are potassium persulfate, ammonium persulfate, sodium persulfate, and 2,2'-azobis(2-amidinopropane) dihydrochloride, because they are readily available and easy to handle. These radical polymerization initiators may be used alone or in combinations of two or more. The above-mentioned radical polymerization initiators may also be used in combination with a reducing agent, such as sodium sulfite, sodium hydrogensulfite, ferrous sulfate, or L-ascorbic acid, and used as a redox polymerization initiator.

The amount of the radical polymerization initiator to be used is, for example, 0.00005 to 0.01 mol per mole of the water-soluble ethylenically unsaturated monomer. When the radical polymerization initiator is used in the above-defined range of amounts, the occurrence of an abrupt polymerization reaction can be avoided, and the polymerization reaction can be completed in an appropriate period of time.

[Internal-Crosslinking Agent]

Examples of the internal-crosslinking agent include those that can crosslink the polymer of the water-soluble ethylenically unsaturated monomer to be used, for example: unsaturated polyesters obtained by reacting polyols, such as diols and triols, e.g., (poly)ethylene glycol ["(poly)" means both cases with and without the prefix "poly"; the same applies below], (poly)propylene glycol, 1,4-butanediol, 1,6-hexanediol, trimethylolpropane, and (poly)glycerin, with unsaturated acids, such as (meth)acrylic acid, maleic acid, and fumaric acid; bisacrylamides, such as N,N-methylenebisacrylamide; di or tri(meth)acrylic acid esters obtained by reacting polyepoxides with (meth)acrylic acid; carbamyl di(meth)acrylates obtained by reacting polyisocyanates, such as tolylene diisocyanate and hexamethylene diisocyanate, with hydroxyethyl (meth)acrylate; compounds having two or more polymerizable unsaturated groups, such as allylated starch, allylated cellulose, diallyl phthalate, N,N',N"-triallyl isocyanurate, and divinylbenzene; polyglycidyl compounds, such as diglycidyl compounds, e.g., (poly)ethylene glycol diglycidyl ether, (poly)propylene glycol diglycidyl ether, and (poly)glycerin diglycidyl ether, and triglycidyl compounds; epihalohydrin compounds, such as epichlorohydrin, epibromohydrin, and $\alpha$-methylepichlorohydrin; compounds having two or more reactive functional groups, such as isocyanate compounds, e.g., 2,4-tolylene diisocyanate and hexamethylene diisocyanate; and oxetane compounds, such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol. Among these internal-crosslinking agents, polyglycidyl compounds are preferably used, diglycidyl ether compounds are more preferably used, and (poly)ethylene glycol diglycidyl ether, (poly)propylene glycol diglycidyl ether, and (poly)glycerin diglycidyl ether are still more preferably used. These internal-crosslinking agents may be used alone or in combinations of two or more.

The amount of the internal-crosslinking agent to be used is preferably 0.000001 to 0.02 mol, more preferably 0.00001 to 0.01 mol, still more preferably 0.00001 to 0.005 mol, and even more preferably 0.00005 to 0.002 mol, per mole of the water-soluble ethylenically unsaturated monomer.

[Hydrocarbon Dispersion Medium]

Examples of the hydrocarbon dispersion medium include $C_{6-8}$ aliphatic hydrocarbons, such as n-hexane, n-heptane, 2-methylhexane, 3-methylhexane, 2,3-dimethylpentane, 3-ethylpentane, and n-octane; alicyclic hydrocarbons, such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons, such as benzene, toluene, and xylene. Among these hydrocarbon dispersion media, n-hexane, n-heptane, and cyclohexane, which are readily industrially available, stable in quality, and inexpensive, are particularly suitably used. These hydrocarbon dispersion media may be used alone or in combinations of two or more. The use of a commercially available mixture of hydrocarbon dispersion media, such as Exxsol Heptane (from Exxon Mobil Corporation; containing 75 to 85% by mass of heptane and its isomeric hydrocarbons), also leads to favorable results.

The amount of the hydrocarbon dispersion medium to be used is preferably 100 to 1500 parts by mass, and more preferably 200 to 1400 parts by mass, per 100 parts by mass of a first-stage water-soluble ethylenically unsaturated monomer, in view of homogeneously dispersing the water-soluble ethylenically unsaturated monomer, and facilitating control of the polymerization temperature. As described below, reversed phase suspension polymerization is performed in one stage (single stage) or two or more multiple stages. The above-mentioned first-stage polymerization refers to the first-stage polymerization reaction in single-stage polymerization or multi-stage polymerization (the same applies below).

[Dispersion Stabilizer]

(Surfactant)

In reversed phase suspension polymerization, a dispersion stabilizer may be used to improve the dispersion stability of the water-soluble ethylenically unsaturated monomer in the hydrocarbon dispersion medium. A surfactant may be used as such a dispersion stabilizer.

Examples of usable surfactants include sucrose fatty acid esters, polyglycerol fatty acid esters, sorbitan fatty acid esters, polyoxyethylene sorbitan fatty acid esters, polyoxyethylene glycerin fatty acid esters, sorbitol fatty acid esters, polyoxyethylene sorbitol fatty acid esters, polyoxyethylene alkyl ethers, polyoxyethylene alkyl phenyl ethers, polyoxyethylene castor oil, polyoxyethylene hydrogenated castor oil, alkyl allyl formaldehyde condensate polyoxyethylene ethers, polyoxyethylene-polyoxypropylene block copolymers, polyoxyethylene polyoxypropyl alkyl ethers, polyethylene glycol fatty acid esters, alkyl glucosides, N-alkyl glyconamides, polyoxyethylene fatty acid amides, polyoxyethylene alkylamines, polyoxyethylene alkyl ether phosphates, and polyoxyethylene alkyl allyl ether phosphates. Among these surfactants, sorbitan fatty acid esters, polyglycerol fatty acid esters, and sucrose fatty acid esters are particularly preferably used, in view of dispersion stability of the monomer. These surfactants may be used alone or in combinations of two or more.

The amount of the surfactant to be used is preferably 0.1 to 30 parts by mass, and more preferably 0.3 to 20 parts by mass, per 100 parts by mass of the first-stage water-soluble ethylenically unsaturated monomer.

(Polymeric Dispersion Agent)

A polymeric dispersion agent may be used in combination with the above-described surfactant, as the dispersion stabilizer to be used in reversed phase suspension polymerization.

Examples of the polymeric dispersion agent include maleic anhydride modified polyethylene, maleic anhydride modified polypropylene, maleic anhydride modified ethylene-propylene copolymers, maleic anhydride modified EPDM (ethylene-propylene-diene terpolymers), maleic anhydride modified polybutadiene, maleic anhydride-ethylene copolymers, maleic anhydride-propylene copolymers, maleic anhydride-ethylene-propylene copolymers, maleic anhydride-butadiene copolymers, polyethylene, polypropylene, ethylene-propylene copolymers, oxidized polyethylene, oxidized polypropylene, oxidized ethylene-propylene copolymers, ethylene-acrylic acid copolymers, ethyl cellulose, and ethyl hydroxyethyl cellulose. Among these polymeric dispersion agents, maleic anhydride modified polyethylene, maleic anhydride modified polypropylene, maleic anhydride modified ethylene-propylene copolymers, maleic anhydride-ethylene copolymers, maleic anhydride-propylene copolymers, maleic anhydride-ethylene-propylene copolymers, polyethylene, polypropylene, ethylene-propylene copolymers, oxidized polyethylene, oxidized polypropylene, and oxidized ethylene-propylene copolymers are particularly preferably used, in view of dispersion stability of the monomer. These polymeric dispersion agents may be used alone or in combinations of two or more.

The amount of the polymeric dispersion agent to be used is preferably 0.1 to 30 parts by mass, and more preferably 0.3 to 20 parts by mass, per 100 parts by mass of the first-stage water-soluble ethylenically unsaturated monomer.

[Other Components]

In the method for producing the water-absorbent resin particles, other components may be optionally added to the aqueous solution containing the water-soluble ethylenically unsaturated monomer, which is then subjected to reversed phase suspension polymerization. Various additives such as thickeners and chain transfer agents can be added as the other components.

By way of example, a thickener may be added to the aqueous solution containing the water-soluble ethylenically unsaturated monomer, which is then subjected to reversed phase suspension polymerization. By thus adding a thickener to adjust the viscosity of the aqueous solution, the median particle size obtained by reversed phase suspension polymerization can be controlled.

Examples of usable thickeners include hydroxyethylcellulose, hydroxypropylcellulose, methylcellulose, carboxymethylcellulose, polyacrylic acid, (partially) neutralized polyacrylic acid, polyethylene glycol, polyacrylamide, polyethyleneimine, dextrin, sodium alginate, polyvinyl alcohol, polyvinylpyrrolidone, and polyethylene oxide. Assuming that the stirring rate during the polymerization is the same, the higher the viscosity of the aqueous solution containing the water-soluble ethylenically unsaturated monomer, the larger the primary particles and/or secondary particles of the resulting particles tend to be.

[Reversed Phase Suspension Polymerization]

To perform reversed phase suspension polymerization, for example, an aqueous monomer solution containing the water-soluble ethylenically unsaturated monomer is dispersed in a hydrocarbon dispersion medium, in the presence of a dispersion stabilizer. Here, as long as the dispersion stabilizer (a surfactant and a polymeric dispersion agent) is added before the beginning of the polymerization reaction, it may be added either before or after the addition of the aqueous monomer solution.

In particular, in view of readily reducing the amount of the hydrocarbon dispersion medium remaining in the resulting water-absorbent resin particles, it is preferred to disperse the aqueous monomer solution in the hydrocarbon dispersion medium in which a polymeric dispersion agent is dispersed and then disperse a surfactant therein, followed by polymerization.

Reversed phase suspension polymerization as described above can be performed in one stage or two or more multiple stages. In view of improving productivity, reversed phase suspension polymerization is preferably performed in two or three stages.

Reversed phase suspension polymerization in two or more multiple stages may be performed as follows: the first stage of reversed phase suspension polymerization is performed; subsequently, the water-soluble ethylenically unsaturated monomer is added to and mixed with the reaction mixture obtained by the first-stage polymerization reaction, and the second and subsequent stages of reversed phase suspension polymerization are performed in the same manner as in the first stage. In each of the second and subsequent stages of reversed phase suspension polymerization, it is preferred to add, in addition to the water-soluble ethylenically unsaturated monomer, a radical polymerization initiator within the above-described range of molar ratios of each component relative to the water-soluble ethylenically unsaturated monomer, based on the amount of the water-soluble ethylenically unsaturated monomer added in each of the second and subsequent stages of reversed phase suspension polymerization, and then perform reversed phase suspension polymerization. In the second and subsequent stages of polymerization, an internal-crosslinking agent may also be optionally added to the water-soluble ethylenically unsaturated monomer.

The reaction temperature during the polymerization reaction is preferably 20 to 110°C, and more preferably 40 to 90°C, in view of allowing the polymerization to proceed quickly to reduce the polymerization time for improved economic efficiency, and readily removing the heat of polymerization to perform the reaction smoothly.

<Surface-Crosslinking Step>

Next, the water-absorbent resin particles of the present invention are obtained by crosslinking the hydrous gel having an internal-crosslinked structure obtained by polymerization of the water-soluble ethylenically unsaturated monomer, by adding a surface-crosslinking agent thereto (surface-crosslinking reaction). The surface-crosslinking reaction is preferably performed in the presence of a surface-crosslinking agent, after the polymerization of the water-soluble ethylenically unsaturated monomer. By thus subjecting the hydrous gel having an internal-crosslinked structure to the surface-crosslinking reaction after the polymerization, it is possible to obtain water-absorbent resin particles having an increased crosslinking density near the surfaces of the water-absorbent resin particles to exhibit improvement in various kinds of performance, such as water-absorption capacity under load.

Examples of the surface-crosslinking agent include compounds with two or more reactive functional groups. Examples include polyols, such as ethylene glycol, propylene glycol, 1,4-butanediol, diethylene glycol, triethylene glycol, trimethylolpropane, glycerin, polyoxyethylene glycol, polyoxypropylene glycol, and polyglycerin; polyglycidyl compounds, such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds, such as epichlorohydrin, epibromohydrin, and $\alpha$-methyl epichlorohydrin; isocyanate compounds, such as 2,4-tolylene diisocyanate and hexamethylene diisocyanate; oxetane compounds, such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol; oxazoline compounds, such as 1,2-ethylenebisoxazoline; carbonate compounds (e.g., alkylene carbonates), such as ethylene carbonate, propylene carbonate, 4,5-dimethyl-1,3-dioxolan-2-one, 4,4-dimethyl-1,3-dioxolan-2-one, 4-ethyl-1,3-dioxolan-2-one, 4-hydroxymethyl-1,3-dioxolan-2-one, 1,3-dioxan-2-one, 4-methyl-1,3-dioxan-2-one, 4,6-dimethyl-1,3-dioxan-2-one, and 1,3-dioxopan-2-one; and hydroxyalkylamide compounds, such as bis[N,N-di($\beta$-hydroxyethyl)]adipamide. Preferred among these surface-crosslinking agents are polyglycidyl compounds, such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether. These surface-crosslinking agents may be used alone or in combinations of two or more.

The amount of the surface-crosslinking agent to be used is preferably 0.00001 to 0.01 mol, more preferably 0.00005 to 0.005 mol, and still more preferably 0.0001 to 0.002 mol, per mole of the total amount of the water-soluble ethylenically unsaturated monomer used for polymerization.

The surface-crosslinking agent may be added as is or as an aqueous solution. Optionally, a solution of the surface-crosslinking agent in a hydrophilic organic solvent may be added. Examples of the hydrophilic organic solvent include lower alcohols, such as methyl alcohol, ethyl alcohol, n-propyl alcohol, and isopropyl alcohol; ketones, such as acetone and methyl ethyl ketone; ethers, such as diethyl ether, dioxane, and tetrahydrofuran; amides, such as N,N-dimethylformamide; and sulfoxides, such as dimethylsulfoxide. These hydrophilic organic solvents may be used alone, in combinations of two or more, or as a solvent mixture with water.

The surface-crosslinking agent may be added at any time after the polymerization reaction of the water-soluble ethylenically unsaturated monomer is substantially completed. The surface-crosslinking agent is preferably added in the presence of 1 to 400 parts by mass of water, more preferably 5 to 200 parts by mass of water, still more preferably 10 to 100 parts by mass of water, even more preferably 20 to 60 parts by mass of water, per 100 parts by mass of the water-soluble ethylenically unsaturated monomer. The amount of water herein refers to the total amount of the water contained in the reaction system and the water optionally used during the addition of the surface-crosslinking agent.

The reaction temperature during the surface-crosslinking reaction is preferably 50 to 250°C, more preferably 60 to 180°C, still more preferably 60 to 140°C, and even more preferably 70 to 120°C. The reaction time of the surface-crosslinking reaction is preferably 1 to 300 minutes, and more preferably 5 to 200 minutes.

<Drying Step>

After reversed phase suspension polymerization is performed as described above, the method may include a drying step of adding external energy, such as heat, to remove the water, the hydrocarbon dispersion medium, and the like by distillation. To remove the water in the hydrous gel after reversed phase suspension polymerization, the system in which the hydrous gel is dispersed in the hydrocarbon dispersion medium is heated to distill the water and the hydrocarbon dispersion medium out of the system by azeotropic distillation. Here, if the distilled hydrocarbon dispersion medium only is returned into the system, continuous azeotropic distillation can be performed. This is preferable in view of preventing deterioration of the resin, because the temperature within the system during drying is maintained at a temperature not higher than the azeotropic temperature with the hydrocarbon dispersion medium. Subsequently, the water and the hydrocarbon dispersion medium are distilled off to obtain water-absorbent resin particles. By controlling the treatment conditions for the drying step after the polymerization to adjust the amount of water to be removed, various kinds of performance of the resulting water-absorbent resin particles can be controlled.

In the drying step, the drying treatment by distillation may be performed under atmospheric pressure or reduced pressure. The drying treatment may also be performed in a stream of nitrogen or the like, in view of improving the drying efficiency. When the drying treatment is performed under atmospheric pressure, the drying temperature is preferably 70 to 250°C, more preferably 80 to 180°C, still more preferably 80 to 140°C, and even more preferably 90 to 130°C. When the drying treatment is performed under reduced pressure, the drying temperature is preferably 40 to 160°C, and more preferably 50 to 110°C.

When the surface-crosslinking step with a surface-crosslinking agent is performed after the polymerization of the monomer by reversed phase suspension polymerization, the drying step by distillation is performed as described above, after the completion of the surface-crosslinking step. Alternatively, the surface-crosslinking step and the drying step may be performed simultaneously.

The water-absorbent resin particles may contain additives suitable for its purpose. Examples of such additives include inorganic powders, surfactants, oxidizing agents, reducing agents, metal chelating agents, radical chain inhibitors, antioxidants, and anti-bacterial agents. For example, when 0.05 to 5 parts by mass of amorphous silica as an inorganic powder is added to 100 parts by mass of the water-absorbent resin particles, the flowability of the water-absorbent resin particles in the absorbent material can be further improved. The additives are preferably hydrophilic or water-soluble.

Examples

The present invention will be hereinafter described in detail with reference to examples and comparative examples. However, the present invention is not limited to the examples.

The below-described water-absorbent resin particles, activated carbons, and the absorbent articles obtained in the examples and comparative examples were evaluated using the tests as described below. Unless otherwise indicated, the measurement was performed in an environment at a temperature of $25 \pm 2$°C and a humidity of $50 \pm 10$%.

[Production of Water-Absorbent Resin Particles]

<Production Example 1>

A 110 mm inner diameter, 2 L volume, cylindrical round-bottomed separable flask with four side wall baffles (baffle width: 7 mm) was prepared which was equipped with a reflux condenser, a dropping funnel, a nitrogen gas inlet tube, and a stirrer. The stirrer was mounted with stirring blades having two stages of four inclined paddle blades surface-treated with a fluororesin and having a blade diameter of 50 mm. In the prepared separable flask, 451.4 g of n-heptane and 1.288 g of sorbitan monolaurate (trade name: NONION LP-20R, HLB value: 8.6, from NOF CORPORATION) were mixed. The mixture in the separable flask was heated to 50°C while stirring with the stirrer to dissolve the sorbitan monolaurate in the n-heptane. The resulting solution was cooled to 40°C.

92.0 g of an 80.5% by mass aqueous solution of acrylic acid (acrylic acid: 1.03 mol) was placed in a 500 mL inner volume Erlenmeyer flask. 147.7 g of a 20.9% by mass aqueous solution of sodium hydroxide was added dropwise into the aqueous solution of acrylic acid in the flask, with external ice-cooling, to partially neutralize the acrylic acid. Subsequently, 0.1011 g (0.374 mmol) of potassium persulfate was added as a water-soluble radical polymerization initiator and dissolved in the aqueous solution to prepare an aqueous monomer solution.

The resulting aqueous monomer solution was added to the separable flask containing the solution containing the sorbitan monolaurate, and the system was sufficiently purged with nitrogen. While stirring with the stirrer at a rotation speed of 700 rpm, the reaction mixture in the separable flask was maintained in a warm water bath at 70°C for 60 minutes, causing the polymerization reaction to proceed.

To the reaction mixture containing the polymer as a hydrous gel produced by the polymerization reaction, a dispersion of 0.092 g of amorphous silica (Tokusil NP-S from Oriental Silicas Corporation) in 100 g of n-heptane was added, and the reaction mixture was stirred for 10 minutes. The separable flask was immersed in an oil bath at 125°C to remove 98.5 g of water out of the system by azeotropic distillation. Then, 4.14 g of a 2% by mass aqueous solution of ethylene glycol diglycidyl ether (ethylene glycol diglycidyl ether: 0.475 mmol) was added as a surface-crosslinking agent, and the flask was maintained at an internal temperature of 80 $\pm$ 2°C for 2 hours to cause the surface-crosslinking reaction to proceed.

The reaction mixture was heated to 125°C to evaporate the water and n-heptane, thus giving a dried product of the polymer particles. This dried product was passed through a sieve with a mesh size of 850 $\mu$m to give 86.3 g of water-absorbent resin particles of Production Example 1. The water-absorbent resin particles had a physiological saline retention amount of 35 g/g, a water-absorption rate of 4 seconds, a median particle size of 360 $\mu$m, a heat release rate of 0.33°C/sec, a physiological saline absorption amount under a load of 4.14 kPa of 11 mL/g, and a BET specific surface area of 0.150 m$^2$/g.

<Production Example 2>

A 11 cm inner diameter, 2 L volume, cylindrical round-bottomed separable flask was prepared which was equipped with a reflux condenser, a dropping funnel, a nitrogen gas inlet tube, and a stirrer with stirring blades having two stages of four inclined paddle blades with a blade diameter of 5 cm. As a hydrocarbon dispersion medium, 293 g of n-heptane was placed in this flask, and 0.736 g of a maleic anhydride-modified ethylene-propylene copolymer (Hi-Wax 1105A from Mitsui Chemicals, Inc.) was added as a polymeric dispersion agent. The mixture was heated with stirring to 80°C to dissolve the dispersion agent and then cooled to 50°C. Separately, in a 300 mL inner volume beaker, 92.0 g (1.03 mol) of an 80.5% by mass aqueous solution of acrylic acid was placed as a water-soluble ethylenically unsaturated monomer, and 147.7 g of a 20.9% by mass aqueous solution of sodium hydroxide was added dropwise, while cooling with ice water, to achieve 75 mol% neutralization. Then, 0.092 g of hydroxyethyl cellulose (HEC AW-15F from Sumitomo Seika Chemicals Co., Ltd.) as a thickener, 0.0736 g (0.272 mmol) of potassium persulfate as a water-soluble radical polymerization agent, and 0.010 g (0.057 mmol) of ethylene glycol diglycidyl ether as an internal-crosslinking agent were added and dissolved to prepare a first-stage aqueous solution. Then, the aqueous solution prepared as above was added to the separable flask and stirred for 10 minutes. Then, a surfactant solution prepared in a 20 mL vial by heating and dissolving 0.736 g of sucrose stearate having an HLB of 3 (Ryoto sugar ester S-370 from Mitsubishi-Kagaku Foods Corporation) as a surfactant in 6.62 g of n-heptane was further added. The system was sufficiently purged with nitrogen while stirring with the stirrer at a rotation speed of 550 rpm. Then, the flask was immersed in a water bath at 70°C and heated, and polymerization was performed for 60 minutes to give a first-stage polymerization slurry.

Separately, in another 500 mL inner volume beaker, 128.8 g (1.43 mol) of an 80.5% by mass aqueous solution of acrylic acid was placed as a water-soluble ethylenically unsaturated monomer, and 159.0 g of a 27% by mass aqueous solution of sodium hydroxide was added dropwise, while cooling with ice water, to achieve 75 mol% neutralization. Then, 0.103 g (0.381 mmol) of potassium persulfate as a water-soluble radical polymerization initiator and 0.0117 g (0.067 mmol) of ethylene glycol diglycidyl ether as an internal-crosslinking agent were added and dissolved to prepare a second-stage aqueous solution.

The separable flask system was cooled to 27°C while stirring with the stirrer at a rotation speed of 1000 rpm. Then, the entire amount of the second-stage aqueous solution was added to the first-stage polymerization slurry, and the system was purged with nitrogen for 30 minutes. Then, the flask was again immersed in a water bath at 70°C and heated, and the polymerization reaction was performed for 60 minutes to give a hydrous gel polymer.

Then, the flask was immersed in an oil bath set at 125 °C to remove 256.1 g of water out of the system, while refluxing the n-heptane, by azeotropic distillation of the water and n-heptane. Then, 4.42 g (0.507 mmol) of a 2% by mass aqueous solution of ethylene glycol diglycidyl ether was added to the flask as a surface-crosslinking agent, and the flask was maintained at 83 °C for 2 hours.

Then, the n-heptane was evaporated at 125 °C to dry the reaction mixture, and the resulting product was subsequently passed through a sieve with a mesh size of 850 $\mu$m to give 230.0 g of water-absorbent resin particles of Production Example 2. The water-absorbent resin particles had a physiological saline retention amount of 41 g/g, a water-absorption rate of 29 seconds, a median particle size of 312 $\mu$m, a heat release rate of 0.14°C/sec, a physiological saline absorption amount under a load of 4.14 kPa of 19 ml/g, and a BET specific surface area of 0.037 m$^2$/g.

<Production Example 3>

The procedure of Production Example 2 was repeated, except that the separable flask system was cooled to 23°C, and then the entire amount of the second-stage aqueous solution was added to the first-stage polymerization slurry, and that the amount of water removed by azeotropic distillation of the n-heptane and water was changed to 275.7 g, to give 218.3 g of the water-absorbent resin particles. The water-absorbent resin particles had a physiological saline retention amount of 52 g/g, a water-absorption rate of 48 seconds, a median particle size of 380 $\mu$m, a heat release rate of 0.09°C/sec, a physiological saline absorption amount under a load of 4.14 kPa of 10 ml/g, and a BET specific surface area of 0.031 m$^2$/g.

[Evaluation of the Water-Absorbent Resin Particles]

<Physiological Saline Retention Amount>

2.0 $\pm$ 0.002 g of the water-absorbent resin particles were weighed into a cotton bag (Cottonbroad No. 60, 100 mm in width $\times$ 200 mm in length), and the cotton bag was placed in a 500 mL beaker. 500 g of a 0.9% by mass aqueous solution of sodium chloride (physiological saline) was poured at once into the cotton bag containing the water-absorbent resin particles so as to prevent formation of unswollen lumps, then the top of the cotton bag was closed with a rubber band, and the cotton bag was allowed to stand for 30 minutes to cause the water-absorbent resin particles to swell. After 30 minutes, the cotton bag was dehydrated for 1 minute using a dehydrator (product number: H-122 from Kokusan Co., Ltd.) set at a centrifugal force of 167 G, and a mass Wd (g) of the cotton bag containing the swollen gel after dehydration was measured. The same operation was performed without adding the water-absorbent resin particles, and an empty mass We (g) of the cotton bag upon wetting was measured. The physiological saline retention amount was calculated according to the following equation:

$$\text{Physiological saline retention amount (g/g)} = [\text{Wd} - \text{We}]/2.0$$

<Measurement of Water-Absorption Rate by the Vortex Method>

50 $\pm$ 0.1 g of physiological saline adjusted to a temperature of 25 $\pm$ 0.2°C in a thermostat was weighed into a 100 mL beaker and stirred with a magnetic stirrer bar (8 mm $\varphi$ $\times$ 30 mm, without a ring) to form a vortex at a rotation speed of 600 rpm. 2.0 $\pm$ 0.002 g of the water-absorbent resin particles were added at once into the physiological saline, and the time (seconds) from the addition of the water-absorbent resin particles to the point at which the vortex on the liquid surface disappeared was measured. The measured time was determined as the water-absorption rate of the water-absorbent resin particles. This water-absorption rate is also referred to as the Vortex method or vortex time.

<Median Particle Size (Particle Size Distribution)>

50 g of the water-absorbent resin particles were used for median particle size (particle size distribution) measurement. JIS standard sieves having mesh sizes of 850 $\mu$m, 500 $\mu$m, 425 $\mu$m, 300 $\mu$m, 250 $\mu$m, 180 $\mu$m, and 150 $\mu$m, and a receiving tray were combined in that order from the top. The water-absorbent resin particles were placed on the top sieve of the combined sieves, and classified by shaking for 20 minutes using a Ro-Tap shaker. After the classification, the particle size distribution was determined by calculating the mass of the water-absorbent resin particles remaining on each sieve as the mass percentage relative to the total mass. With regard to this particle size distribution, the mass percentage of the water-absorbent resin particles remaining on each sieve was integrated in descending order of particle size. In this manner, the relationship between the sieve mesh size and the cumulative value of the mass percentage of the water-absorbent resin particles remaining on each sieve was plotted on logarithmic probability paper. The plots on the probability paper were connected with straight lines, and a particle size equivalent to a 50% by mass cumulative mass percentage was determined as the median particle size.

<Physiological Saline Absorption Amount Under a Load of 4.14 kPa>

Physiological saline absorption amount under a load of 4.14 kPa (water absorption amount under load) was measured using the measurement apparatus as schematically shown in Fig. 2. Two measurements were made for one type of water-absorbent resin particles, and the average value was obtained. The measurement apparatus includes a burette unit 1, a clamp 3, a conduit 5, a stand 11, a measurement table 13, and a measurement unit 4 placed on the measurement table 13. The burette unit 1 has a graduated burette tube 21, a rubber stopper 23 for sealing the upper opening of the burette tube 21, a cock 22 connected to the lower end of the burette tube 21, an air inlet tube 25 connected to a lower portion of the burette tube 21, and a cock 24. The burette unit 1 is fixed by the clamp 3. The flat measurement table 13 has a through hole 13a with a diameter of 2 mm formed in the central portion thereof, and is supported by the variable-height stand 11. The through hole 13a of the measurement table 13 and the cock 22 of the burette unit 1 are connected through the conduit 5. The conduit 5 has an inner diameter of 6 mm.

The measurement unit 4 includes a cylinder 31 made of plexiglas, a polyamide mesh 32 bonded to one opening of the cylinder 31, and a weight 33 vertically movable within the cylinder 31. The cylinder 31 is placed on the measurement table 13 with the polyamide mesh 32 therebetween. The cylinder 31 has an inner diameter of 20 mm. The polyamide mesh 32 has a mesh size of 75 $\mu$m (200 mesh). The weight 33 has a diameter of 19 mm and a mass of 119.6 g and, as described later, can apply a load of 4.14 kPa (0.6 psi) to water-absorbent resin particles 10a uniformly disposed on the polyamide mesh 32.

First, the cock 22 and the cock 24 of the burette unit 1 were closed, and 0.9% by mass physiological saline adjusted to 25°C was placed in the burette tube 21 through the upper opening of the burette tube 21. Subsequently, the upper opening of the burette tube 21 was sealed with the rubber stopper 23, and then the cock 22 and the cock 24 were opened. The conduit 5 was filled with the 0.9% by mass physiological saline 50 to prevent air bubbles from entering. The height of the measurement table 13 was adjusted so that the height of the surface of the 0.9% by mass physiological saline reached into the through hole 13a was equal to the height of the upper surface of the measurement table 13. After the adjustment, the height of the surface of the 0.9% by mass physiological saline 50 in the burette tube 21 was read on the graduations of the burette tube 21, and this position was determined as a zero point (reading at 0 seconds).

In the measurement unit 4, 0.10 g of the water-absorbent resin particles 10a were uniformly disposed on the polyamide mesh 32 in the cylinder 31, the weight 33 was disposed on the water-absorbent resin particles 10a, and the cylinder 31 was installed such that the central portion thereof aligned with the conduit opening at the central portion of the measurement table 13. An amount of reduction Wc (mL) in the physiological saline in the burette tube 21 at 60 minutes after the beginning of absorption of the physiological saline by the water-absorbent resin particles 10a through the conduit 5 (that is, the amount of the physiological saline absorbed by the water-absorbent resin particles 10a) was read, and the physiological saline absorption capacity under a load of 4.14 kPa of the water-absorbent resin particles 10a was calculated using the following equation:

Physiological saline absorption capacity (mL/g) under a load of 4.14 kPa = We (mL)/mass (g) of the water-absorbent resin particles

<Heat Release Rate of the Water-Absorbent Resin Particles>

The following measurement was performed in an environment at a temperature of 25 $\pm$ 2°C and a humidity of 50 $\pm$ 10%. A 300 mL volume (inner diameter: 65 mm, height: 120 mm) stainless steel Dewar bottle (D-301 from Thermos) with a cork stopper was prepared. A drip-proof digital thermometer (CT-422WR from CUSTOM Corporation) had been inserted at a position 18 mm in the circumference from the center of the cork stopper, with its measurement tip being fixed at a position 5 mm above the bottom of the stainless steel Dewar bottle. A magnetic stirrer bar (30 mm $\times$ 8 mm $\varphi$) and 50.00 g of physiological saline at 24.9°C were placed in the stainless steel Dewar bottle, and the physiological saline was stirred with the magnetic stirrer at 600 rpm. While stirring the physiological saline, 10.0000 $\pm$ 0.0002 g of the water-absorbent resin particles were added into the stainless steel Dewar bottle, which was then covered with the cork stopper with the thermometer. A time $t_1$ (seconds) required for the temperature displayed on the thermometer to increase by 2.0°C and reach 26.9°C after the addition of water-absorbent resin particles was measured. The value calculated from the measured value using the following equation was defined as the heat release rate:

$$\text{Heat release rate (°C/sec)} = 2.0 \ (°C)/t_1 \ (sec)$$

<BET Specific Surface Area of the Water-Absorbent Resin Particles>

The water-absorbent resin particles to be measured were adjusted to a particle size such that they passed through a sieve with a mesh size of 400 $\mu$m and retained on a sieve with a mesh size of 300 $\mu$m, and then used for specific surface area measurement. Subsequently, 10 g of the classified sample was dispersed in 100 g of ethanol and washed for 5 minutes in an ultrasonic cleaner (US-103 from SND Co., Ltd.) and then filtered through a sieve with a mesh size of 300 $\mu$m. Two more washing operations were repeated in the same manner to give a measurement sample washed a total of three times. This sample was dried under degassing conditions of evacuation with heating at 100°C for 16 hours. Then, an adsorption isotherm was measured at a temperature of 77 K using a specific surface area analyzer (AUTOSORB-1 from Quantachrome), by the method using krypton gas as the adsorption gas, and the specific surface area was determined from a multi-point BET plot as the BET specific surface area of the water-absorbent resin particles.

[Preparation of Activated Carbons]

<Activated Carbon A>

An activated carbon (product name: FP-3 from Osaka Gas Chemicals Co., Ltd.) was prepared having a BET specific surface area of 1619 $m^2$/g, a median particle size of 38 $\mu$m, a residue on ignition of 0.1%, a loss on drying of 0.8%, an iodine adsorption amount of 1560 mg/g, a pH of 7.1, and a crushed shape. This activated carbon was designated as activated carbon A.

[Evaluation of Activated Carbon]

<Median Particle Size (Laser Diffraction) of Activated Carbon>

The median particle size (D50 (median size), volume-based) of the activated carbon used was measured using a laser diffraction particle size distribution analyzer (SALD-2300 from Shimadzu Corporation).

<BET Specific Surface Area of Activated Carbon>

0.1 g of the activated carbon to be measured was dried under degassing conditions of evacuation with heating at 60°C for 24 hours, using a pretreatment apparatus (BELPREP VAC II from MicrotracBel Corporation). Then, an adsorption isotherm was measured at a temperature of 77 K using a specific surface area analyzer (BELSORP MINI II from MicrotracBel Corporation), by the method using nitrogen gas as the adsorption gas, and the specific surface area was determined from a multi-point BET plot as the BET specific surface area of the activated carbon.

[Production of Absorbent Articles]

<Example 1>

A tissue (12 cm $\times$ 11 cm, 22 $g/m^2$) was overlaid with a sheet of molded pulp of the same size (12 cm $\times$ 11cm, 189 $g/m^2$). 3.75 g (284 $g/m^2$) of the water-absorbent resin particles produced in Production Example 1 were uniformly distributed on the sheet of molded pulp, and subsequently 0.0113 g (0.86 $g/m^2$) of activated carbon A was uniformly distributed on the water-absorbent resin particles. The activated carbon A was further overlaid with the above-mentioned sheet of molded pulp, the above-mentioned tissue, and a liquid-permeable sheet of the same size (made of 50% of polyethylene and 50% of polypropylene; 12 cm $\times$ 11 cm, 21 $g/m^2$) in this order, and then the resulting laminate was pressed by applying a load of 284 kPa to the entire laminate for 30 seconds to give a laminate including the absorbent material. A liquid-impermeable sheet of the same size (made of polyethylene; 12 cm $\times$ 11 cm, 39 $g/m^2$) was laminated to the lower side of the laminate to produce an absorbent article of Example 1.

<Example 2>

The procedure of Example 1 was repeated, except that 3.75 g (284 $g/m^2$) of the water-absorbent resin particles produced in Production Example 2, instead of 3.75 g (284 $g/m^2$) of the water-absorbent resin particles produced in Production Example 1, were uniformly distributed, to give an absorbent article of Example 2.

<Example 3>

A tissue (12 cm $\times$ 11 cm, 22 $g/m^2$) was overlaid with a sheet of molded pulp of the same size (12 cm $\times$ 11cm, 189 $g/m^2$). 3.75 g (284 $g/m^2$) of the water-absorbent resin particles produced in Production Example 2 were uniformly distributed on the sheet of molded pulp, and the water-absorbent resin particles were overlaid with the above-mentioned sheet of molded pulp and the above-mentioned tissue in this order. Subsequently, 0.0113 g (0.86 $g/m^2$) of activated carbon A was uniformly distributed on the tissue. The activated carbon A was overlaid with a liquid-permeable sheet of the same size (made of 50% of polypropylene and 50% of polyethylene; 12 cm $\times$ 11 cm, 21 $g/m^2$), and then the resulting laminate was pressed by applying a load of 284 kPa to the entire laminate for 30 seconds to give a laminate including the absorbent material. A liquid-impermeable sheet of the same size (made of polyethylene; 12 cm $\times$ 11 cm, 39 $g/m^2$) was laminated to the lower side of the laminate to produce an absorbent article of Example 3.

<Comparative Example 1>

The procedure of Example 1 was repeated, except that 3.75 g (284 g/m$^2$) of the water-absorbent resin particles produced in Production Example 3, instead of 3.75 g (284 g/m$^2$) of the water-absorbent resin particles produced in Production Example 1, were uniformly distributed, to give an absorbent article of Comparative Example 1.

<Comparative Example 2>

The procedure of Example 1 was repeated, except that 3.75 g (284 g/m$^2$) of the water-absorbent resin particles produced in Production Example 1 were not distributed, to give an absorbent article of Comparative Example 2.

<Comparative Example 3>

The procedure of Example 2 was repeated, except that activated carbon A was not distributed, to give an absorbent article of Comparative Example 3.

[Evaluation of Absorbent Articles]

<Deodorization Test>

The deodorizing performance of each absorbent article was evaluated using the following procedure. First, a stainless steel petri dish ($\varphi$ 120 mm $\times$ 25 mm) was prepared, and the produced absorbent article was placed therein. 50 mL of test human urine was added thereto (the test human urine was prepared by collecting urine from seven adults aged 40 to 60 and mixing, and used within the day). The stainless steel petri dish containing the absorbent article was placed in a 10 L polyester sampling bag (PAAAK10 from GL Sciences Inc.), and the bag was sealed by pressure-bonding with a heat sealer (model number: FI-450-5 from Fuji Impulse Co., Ltd). A 1000 mL volume syringe was used to remove the gas phase inside the sampling bag and instead inject 5 L of dry air purified by passing through an activated carbon layer. Then, the sampling bag was placed and stored in a constant temperature and humidity chamber (LHU-113 from Espec Corporation) set at a temperature of 36 $\pm$ 1°C and a humidity of 60 $\pm$ 5%. After 1 hour, the sampling bag was removed from the constant temperature and humidity chamber, and 1000 mL of the gas phase was collected into a previously degassed 1 L sampling bag (PAAAK1 from GL Sciences Inc.). The same procedure was also performed for the absorbent article not blended with activated carbon (Comparative Example 3). The gas phase was collected, and the odor of this gas phase was used as a control.

Six panelists were asked to smell the odor of the gas phase collected from each of the absorbent articles of Examples 1 to 3, the absorbent articles of Comparative Examples 1 and 2, and the absorbent article of Comparative Example 3 as the control, and evaluate according to the following evaluation criteria. When the average value was 2 or less, the absorbent article was evaluated as having a deodorizing effect. The average value and evaluation result is shown in Table 1.

5: Increased odor compared to the control
4: No change in odor compared to the control
3: Slightly decreased odor compared to the control
2: Decreased odor compared to the control
1: Significantly decreased odor compared to the control
0: No odor

[Table 1]

| Table 1 | Water-absorbent resin partic | | | | Activated carbon | | Absorbent article | | |
|---|---|---|---|---|---|---|---|---|---|
| | Physiological saline absorption amount (g/g) | Physiological saline absorption amount (mL/g) under a load of 4.14 kPa | Median particle size (μm) | Heat release rate (°C/sec) | Median particle size (μm) | Amount Present (g/m²) | Lamination Structure (from the human urine supply side to the outside) | Deodorizing effect | |
| | | | | | | | | Score | Evaluation |
| Ex. 1 | 35 | 11 | 360 | 0.33 | 38 | 0.86 | liquid-permeable sheet/ tissue/absorbent material (pulp/ activated carbon/ water-absorbent resin particles/ pulp)/tissue/ liquid-impermeable sheet | 1.5 | Yes |
| Ex. 2 | 41 | 19 | 312 | 0.14 | 38 | 0.86 | liquid-permeable sheet/tissue/ absorbent material (pulp/ activated carbon/ water-absorbent resin particles/ pulp)/tissue/ liquid-impermeable sheet | 1.8 | Yes |
| Ex. 3 | 41 | 19 | 312 | 0.14 | 38 | 0.86 | liquid-permeable sheet/activated carbon/tissue/ absorbent material (pulp/ water-absorbent resin partides/ pulp)/tissue/ liquid-impermeable sheet | 1.8 | Yes |
| Comp. Ex. 1 | 52 | 10 | 380 | 0.09 | 38 | 0.86 | liquid-permeable sheet/tissue/ absorbent material (pulp/ activated carbon/ water-absorbent resin particles/ pulp)/tissue/ liquid-impermeable sheet | 2.3 | No |

(continued)

| Table 1 | Water-absorbent resin partic | | | | Activated carbon | | Absorbent article | | |
|---|---|---|---|---|---|---|---|---|---|
| | Physiological saline absorption amount (g/g) | Physiological saline absorption amount (mL/g) under a load of 4.14 kPa | Median particle size (μm) | Heat release rate (°C/sec) | Median particle size (μm) | Amount Present (g/m²) | Lamination Structure (from the human urine supply side to the outside) | Deodorizing effect | |
| | | | | | | | | Score | Evaluation |
| Comp. **Ex.** 2 | - | - | - | - | 38 | 0.86 | liquid-permeable sheet/tissue/ absorbent material (pulp activated carbon/ pulp)/rissue/ liquid- impermeable sheet | 2.3 | No |
| Comp. Ex. 3 | 41 | 19 | 312 | 0.14 | - | 0.00 | liquid-permeable sheet/tissue/ absorbent material (pulp/ water-absorbent resin particles/ pulp)/tissue/ liquid- impermeable sheet | 4.0 | No |

Reference Signs List

1: burette unit
3: clamp
4: measurement unit
5: conduit
10: absorbent material
10a: water-absorbent resin particles
10b: fiber layer
10c: activated carbon
11: stand
13: measurement table
13a: through hole
15: nylon mesh sheet
20: core wrap
21: burette tube
22: cock
23: rubber stopper
24: cock
25: air inlet tube
30: liquid-permeable sheet
31: cylinder
32: polyamide mesh
33: weight

40: liquid-impermeable sheet
100: absorbent article

## Claims

1. An absorbent article comprising at least a liquid-permeable sheet; a liquid-impermeable sheet; and an absorbent material and an activated carbon disposed between the liquid-permeable sheet and the liquid-impermeable sheet,

   wherein the absorbent material comprises water-absorbent resin particles, and
   the water-absorbent resin particles have a heat release rate of 0.10°C/sec or more and 1.00°C/sec or less as measured by the following method of measuring the heat release rate:

   (Method of Measuring the Heat Release Rate)
   50 g of physiological saline at 24.9°C is added into a 300 mL volume, cylindrical thermally insulated container and stirred at 600 rpm;
   while stirring the physiological saline, 10 g of the water-absorbent resin particles are further added into the thermally insulated container, and the thermally insulated container is closed with a cork stopper having a thermometer; and
   a time required for the temperature of the physiological saline to increase by 2.0°C after the addition of the water-absorbent resin particles is measured, and a value of heat release temperature per second is calculated as the heat release rate.

2. The absorbent article according to claim 1, wherein when the absorbent article is viewed in plan view, the activated carbon has a basis weight per unit area of 0.001 $g/m^2$ or more and 6.000 $g/m^2$ or less in a region where the activated carbon is contained.

3. The absorbent article according to claim 1, wherein when the absorbent article is viewed in plan view, the activated carbon has a basis weight per unit area of 0.025 $g/m^2$ or more and 7.000 $g/m^2$ or less in that region.

4. The absorbent article according to any one of claims 1 to 3, wherein the activated carbon has a median particle size of 1 $\mu$m or more and 100 $\mu$m or less.

FIG. 1

FIG. 2

## INTERNATIONAL SEARCH REPORT

| | International application No. |
|---|---|
| | **PCT/JP2022/043885** |

### A. CLASSIFICATION OF SUBJECT MATTER

*A61F 13/15*(2006.01)i; *A61F 13/53*(2006.01)i
FI: A61F13/53 300; A61F13/15 141

According to International Patent Classification (IPC) or to both national classification and IPC

### B. FIELDS SEARCHED

Minimum documentation searched (classification system followed by classification symbols)

A61F13/15; A61F13/53

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | JP 2003-102784 A (TOYO BOSEKI) 08 April 2003 (2003-04-08)<br>paragraphs [0004]-[0040] | 1-4 |
| Y | JP 2018-536495 A (THE PROCTER & GAMBLE COMPANY) 13 December 2018 (2018-12-13)<br>paragraphs [0025]-[0026], [0033], [0085] | 1-4 |
| Y | JP 2011-239934 A (DAIO PAPER CORP) 01 December 2011 (2011-12-01)<br>paragraphs [0037]-[0038] | 1-4 |
| Y | JP 2015-70995 A (LIVEDO CORP) 16 April 2015 (2015-04-16)<br>paragraphs [0008], [0014], [0017] | 1-4 |
| Y | JP 2010-194254 A (KAO CORP) 09 September 2010 (2010-09-09)<br>paragraphs [0021], [0024], [0045]-[0046] | 1-4 |
| A | JP 2020-141874 A (UNICHARM CORP) 10 September 2020 (2020-09-10)<br>paragraphs [0054]-[0056], [0063], [0068] | 1-4 |

☐ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **14 February 2023** | **28 February 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/043885**

| Patent document cited in search report | | | Publication date (day/month/year) | Patent family member(s) | | | Publication date (day/month/year) |
|---|---|---|---|---|---|---|---|
| JP | 2003-102784 | A | 08 April 2003 | (Family: none) | | | |
| JP | 2018-536495 | A | 13 December 2018 | US | 2017/0165134 | A1 | |
| | | | | paragraphs [0033]-[0034], [0039], [0092] | | | |
| | | | | WO | 2017/100440 | A1 | |
| | | | | CN | 108366889 | A | |
| JP | 2011-239934 | A | 01 December 2011 | (Family: none) | | | |
| JP | 2015-70995 | A | 16 April 2015 | (Family: none) | | | |
| JP | 2010-194254 | A | 09 September 2010 | (Family: none) | | | |
| JP | 2020-141874 | A | 10 September 2020 | WO | 2020/179884 | A1 | |
| | | | | CN | 111787895 | A | |

Form PCT/ISA/210 (patent family annex) (January 2015)

**REFERENCES CITED IN THE DESCRIPTION**

**Patent documents cited in the description**

- JP 2001323155 A

- WO 2011086843 A